# EUROPÄISCHE PATENTSCHRIFT

(11) **EP 3 636 233 B1**
(45) Veröffentlichungstag und Bekanntmachung des Hinweises auf die Patenterteilung: **07.06.2023**
(21) Anmeldenummer: 18200151.1
(22) Anmeldetag: 12.10.2018
(51) Int. Cl.: A61F 13/02

(54) **FEUCHTE WUNDAUFLAGE MIT KLEBERAND**
WET WOUND DRESSING HAVING AN ADHESIVE EDGE
PANSEMENT HUMIDE À BORD DE COLLAGE

(43) Veröffentlichungstag der Anmeldung: 15.04.2020
(73) Patentinhaber: IVF Hartmann AG, 8212 Neuhausen (CH)
(72) Erfinder: Marz, Jacqueline, 78315 Radolfzell (DE); Rothmaier, Markus, 8308 Illnau (CH)
(74) Vertreter: Matzdorf, Thorsten

(56) Entgegenhaltungen:
- WO-A1-2009/000416
- WO-A2-2009/019229
- DE-A1- 19 916 523
- DE-A1-102010 020 050
- US-A- 5 792 089

## Beschreibung

Die vorliegende Erfindung betrifft eine Wundauflage zur Wundbehandlung im feuchten oder feuchtnassen Milieu umfassend einen Saug-/Spülkörper, der herstellerseitig mit einer salzhaltigen wässrigen Lösung beaufschlagt ist und ein flächiges Trägermaterial, welches auf der wundabgewandten Seite des Saug-/Spülkörpers angeordnet ist und den Saug-/Spülkörper so überragt, dass ein allseitig den Saug-/Spülkörper umlaufender Rand gebildet wird, der auf der wundzugewandten Seite mit einer Schicht aus einem hautverträglichen Silikonklebstoff versehen ist, sowie eine Verpackung für eine derartige Wundauflage, ein Herstellungsverfahren für ein Wundversorgungsprodukt enthaltend eine Wundauflage in einer Verpackung, und ein Sortiment mehrerer Wundauflagen.

Wundauflagen zur Wundbehandlung im feuchten oder feuchtnassen Milieu sind aus dem Stand der Technik bekannt. So offenbaren beispielsweise die Dokumente WO2011/141454, EP 0 594 034B1 oder WO2016/156619 der Anmelderin eine wundkissenartige oder kompressenartige Wundauflage, die auf eine Wunde aufbringbar ist oder auch zum Austamponieren tiefer Wunden verwendet werden kann. Der Saug-/Spülkörper wird herstellerseitig, insbesondere bis zur Sättigung mit einer salzhaltigen wässrigen Lösung beaufschlagt, welche ein im Saug-/Spülkörper enthaltenes superabsorbierendes Material quellen und in einen gelartigen Zustand übergehen lässt. Dies verleiht dem Saug-/Spülkörper eine duale Funktion bei Wunden mit starker Exsudation. Wundsekrete werden einschließlich ihrer kritischen Bestandteile wie Keime durch den Saug-/Spülkörper aktiv aufgenommen und darin gehalten, wobei der Saug-/Spülkörper im Gegenzug die salzhaltige wässrige Lösung an die Wunde abgibt und somit ein feuchtes Wundmilieu schafft oder unterstützt. Hierdurch wird die Wundreinigung und eine positive Wundkonditionierung unterstützt und somit die Heilung positiv beeinflusst. Man spricht hier von interaktiver Nasstherapie, die insbesondere bei schlecht heilenden Wunden, bei klinisch manifest infizierten Wunden oder bei chronischen Wunden mit unterschiedlicher Genese, wie diabetisches Gangrän, Dekubitalulcus oder Ulcus cruris bevorzugt Anwendung findet.

Nachteil der in den erwähnten Dokumenten beschriebenen Wundauflagen ist, dass sie aufgrund der Feuchtigkeit keine eigene Adhäsivität aufweisen und so unbedingt durch einen Sekundärverband auf der Haut des Patienten fixiert werden müssen. Dieses Problem soll dadurch behoben werden, dass die Wundauflage mit einer Trägerschicht versehen wird, die die Wundauflage wenigstens teilweise überragt und am des Wundkissen überragenden Rand mit einem hautfreundlichen Klebstoff versehen ist. So offenbart beispielsweise das Dokument WO2009/000416 einen Wundverband umfassend ein aktiviertes oder vor der Wundbehandlung zu aktivierendes Wundkissen zur Wundbehandlung im feuchten Milieu mit einem flächigen Trägermaterial, das das Wundkissen zumindest bereichsweise überragt, wobei das Trägermaterial auf der wundzugewandten Seite zumindest bereichsweise mit einer Klebeschicht versehen ist zum Fixieren des Wundverbandes an einem Träger. WO2017/114825 offenbart eine Wundauflage zur Wundbehandlung insbesondere im feuchten oder feuchtnassen Milieu, mit einem Wundkissen, wobei das Wundkissen herstellerseitig mit einer salzhaltigen wässrigen Lösung, insbesondere Ringerlösung, beaufschlagt und in einer Umverpackung aufgenommen sein kann, wobei das Wundkissen herstellerseitig zur Abgabe an den Verbraucher in einer eine Ebene bildenden nicht gefalteten Konfiguration dargeboten ist. Dabei zeichnet sich die Wundauflage dadurch aus, dass ein über das Wundkissen hinausragender Kleberand auf die Rückseite gefaltet ist und dort mit einer Abdeckschicht bedeckt ist.

Besondere Schwierigkeiten treten dabei auf, dass einerseits der klebende Randbereich vor Kontakt mit Schmutz, Flüssigkeit oder Wasserdampf geschützt werden muss, damit er vor Applikation der Wundauflage keine Klebkraft einbüßt. Andererseits muss ermöglicht werden, dass nach Herstellung der trockenen Wundauflage die Beaufschlagung des Saug-/Spülkörpers mit einer salzhaltigen wässrigen Lösung in einer produktionstechnisch vorteilhaften Weise ermöglicht wird.

Die vorliegende Erfindung gemäß Anspruch 1 löst diese Probleme.

Eine Wundauflage gemäß der vorliegenden Erfindung umfasst a) einen Saug-/Spülkörper, der herstellerseitig mit einer salzhaltigen wässrigen Lösung, insbesondere Ringerlösung beaufschlagt ist, wobei die Lösung vorzugsweise eine Substanz mit antimikrobieller Wirkung enthält, b) ein flächiges Trägermaterial, welches auf der wundabgewandten Seite des Saug-/Spülkörpers angeordnet ist und den Saug-/Spülkörper so überragt, dass ein allseitig den Saug-/Spülkörper umlaufender Rand gebildet wird, der auf der wundzugewandten Seite mit einer Schicht aus einem hautverträglichen Silikonklebstoff versehen ist, und c) einer ersten Schutzschicht, die auf der wundzugewandten Seite die Schicht aus einem hautverträglichen Silikonklebstoff abdeckt, wobei die Wundauflage eine zweite Schutzschicht aufweist, die auf der wundabgewandten Seite des Trägermaterials angeordnet ist, wobei sowohl die erste als auch die zweite Schutzschicht das Trägermaterial ganzseitig überragen, wobei die erste Schutzschicht eine Öffnung bildet, die den Saug-/Spülkörper unbedeckt lässt. Die erste und zweite Schutzschicht besteht aus einem flüssigkeits- und wasserdampfundurchlässigen Material, welches Polyester, Polyamid oder Polypropylen umfasst. Die erste und zweite Schutzschicht schützt die Schicht aus hautverträglichem Silikonklebstoff vor Kontakt mit Flüssigkeit und Wasserdampf.

Bei der vorerwähnten Ringerlösung handelt es sich typischerweise um eine wässrige Lösung mit Natriumchlorid, Kaliumchlorid und Calciumchlorid (insbesondere 8,6 g NaCl, 0,3 g KCl und 0,33 g CaCl₂ je Liter).

Die Wechselintervallszeit, d.h. die Verwendungszeit einer Wundauflage bis zum nächsten Verbandwechsel, soll wenigstens 24 h betragen, wobei Bestrebungen bestehen, die Wechselintervallszeit zu erhöhen, insbesondere auf 48 bis 72 h. Dies wäre aus betriebswirtschaftlichen Gesichtspunkten, aber auch aus Gründen einer durch häufige Verbandwechsel verursachten Störung der Wundheilung wünschenswert.

Bei dem Saug-/Spülkörper handelt es sich um ein Wundkissen, welches in der Lage ist Flüssigkeiten zu absorbieren. Dabei kann das Wundkissen aus jedem für diesen Zweck geeigneten Material bestehen und beispielsweise einen Schaum, absorbierende Fasern, ein textiles Material, ein Gel oder absorbierende Partikel enthalten. Insbesondere handelt es sich bei dem Wundkissen um ein nicht-wundverklebendes Wundkissen, das eine Hülle aus einem nicht-wundverklebenden Polymermaterial umfasst. Diese Hülle kann weiterhin bevorzugt aus einem nicht-wundverklebenden Gestrick, Gewirk oder Gewebe bestehen, das weiterhin bevorzugt aus einem hydrophoben Fasermaterial besteht. Insbesondere kann die Hülle ein Gestrick, Gewirk oder Gewebe aus einem hydrophoben Polyethylen-, Polypropylen-, Polyester- oder Viskose-Polymermaterial bestehen. Durch die Ausgestaltung als Gestrick, Gewirk oder Gewebe kann die Hülle in einer oder mehreren Richtungen gedehnt oder verformt werden, ohne dass sie sich von selbst wieder zusammenzieht oder ausrichtet. Die Oberfläche einer derartigen Hülle gleicht sich zudem passgenau an die Oberfläche der zu behandelnden Haut oder Wunde an.

Bevorzugt wird ein Saug-/Spülkörper eingesetzt, der eine Mischung aus absorbierenden Fasern und superabsorbierenden Partikeln enthält und von einer Umhüllung umgeben ist.

Bevorzugt umfasst die Umhüllung auf der wundabgewandten Seite der Wundauflage eine Faservliesschicht, welche die wundabgewandte äußere Sichtseite der Wundauflage bildet. Auf der wundzugewandten Seite dieser Faservliesschicht ist eine flüssigkeitsundurchlässige Kunststofffilmschicht angeordnet, und die Faservliesschicht, die Kunststofffilmschicht, der Saug-/Spülkörper und die Schicht aus textilem Flächenmaterial sind nicht flächenhaft miteinander verbunden, sondern liegen mit ihren Flachseiten lose und verschieblich gegeneinander an. Dabei sind die Faservliesschicht, die Kunststofffilmschicht, der Saug-/Spülkörper und die Schicht aus textilem Flächenmaterial nur entlang ihres Umfangsrands durch eine Fügeverbindung miteinander verbunden.

Diese nur randseitig vorgesehene Fügeverbindung zwischen den Schichten kann in vorteilhafter Weise von einer Ultraschallschweißverbindung oder einer Laserschweißverbindung gebildet sein. Die Ultraschall- oder Laserschweißverbindung kann eine Aufeinanderfolge von diskreten Ultraschallschweißstellen umfassen. Diese Ultraschallschweißstellen können aber auch derart quasi kontinuierlich aufeinanderfolgend angeordnet sein, dass visuell der Eindruck einer kontinuierlich durchgehenden Verbindungslinie entsteht.

Die erwähnte sichtseitige Faservliesschicht kann aus thermoplastischem Fasermaterial, insbesondere aus Polyolefin, insbesondere aus Polypropylen, gebildet sein.

Das Flächengewicht der Faservlieslage auf der wundabgewandten Seite der Wundauflage beträgt vorzugsweise 15-100 g/m², bevorzugt 20-60 g/m², besonders bevorzugt 25-40 g/m².

Weiter erweist es sich als vorteilhaft, wenn die Dichte der Faservliesschicht auf der wundabgewandten Seite der Wundauflage 10-1000 kg/m³, bevorzugt 50-250 kg/m³, besonders bevorzugt 100-150 kg/m³ beträgt.

Weiter erweist es sich als vorteilhaft, wenn die flüssigkeitsundurchlässige Kunststofffilmschicht aus einem thermoplastischen Material, insbesondere aus Polyolefin, insbesondere aus Polypropylen, gebildet ist.

Weiter erweist es sich als vorteilhaft, wenn die flüssigkeitsundurchlässige Kunststofffilmschicht ein Flächengewicht von 5-100g/m², bevorzugt 8-50 g/m², besonders bevorzugt 10-25 g/m² und eine Dicke von 5-100 µm, bevorzugt 8-50 µm, besonders bevorzugt 10-25 µm aufweist.

Es erweist sich als vorteilhaft, wenn der faservliesbasierte Saug-/Spülkörper cellulosische Fasern, insbesondere eine Mischung aus cellulosischen Fasern und thermoplastischen Fasern, insbesondere Polyolefin-Fasern, insbesondere Polypropylen-Fasern oder Polypropylen/Polyethylen-Fasern, umfasst.

Dabei beträgt das Flächengewicht des Faseranteils des Saug-/Spülkörpers vorteilhafterweise 20 bis 500 g/m², bevorzugt 30-300 g/m², besonders bevorzugt 30-200 g/m², insbesondere 30-150 g/m².

Es erweist sich als vorteilhaft, wenn die Dichte des Faseranteils des Saug/Spülkörpers 20-500 kg/m³, bevorzugt 30-300 kg/m³, besonders bevorzugt 50-200 kg/m³ beträgt. Weiter erweist es sich als vorteilhaft, wenn die Schicht aus textilem Flächenmaterial aus einem thermoplastischen Material, insbesondere aus Polyolefin, insbesondere aus Polypropylen, gebildet ist.

Es erweist sich als vorteilhaft, wenn die wundzugewandte Schicht aus einem textilen Flächenmaterial auf der wundzugewandten Seite eine außenseitig partiell und strukturiert aufgebrachte atraumatisch wirkende Beschichtung, vorzugsweise aus Silikon, mit einem Bedeckungsgrad von höchstens 70 % aufweist. Weiter erweist es sich als vorteilhaft, wenn der Bedeckungsgrad der partiell und strukturiert aufgebrachten atraumatisch wirkenden Beschichtung 20 - 70 %, insbesondere 25 - 50 %, insbesondere 30 - 40 % beträgt.

Es erweist sich als weiter vorteilhaft, wenn die partiell und strukturiert aufgebrachte atraumatisch wirkende Beschichtung streifenförmig ausgebildet ist. Die Streifen können dabei linear erstreckt sein. Sie verlaufen vorzugsweise parallel oder äquidistant zueinander. Die Breite eines Streifens beträgt vorteilhafterweise 1 bis 3 mm. Der Abstand der Streifen voneinander beträgt vorteilhafterweise 4 bis 8 mm, insbesondere 4 bis 6 mm.

Weiter erweist es sich als vorteilhaft, wenn die Wundauflage derart ausgebildet ist, dass das superabsorbierende Material anionisch ist und negative Gruppen aufweist und dass die wässrige Lösung eine Substanz mit antimikrobieller Wirkung umfasst, bei der es sich um Silberkationen, Biguanid oder Biguanid-Derivate, Polyguanidine, N-Octyl-1-[10-(4-Octyliminopyridin- 1-yl)decyl]pyridin-4-imin (Octenidin), quartäre Ammoniumverbindungen, Triazine oder die Ammoniumverbindung Taurolidin handelt, und dass diese Substanz mit antimikrobieller Wirkung bei pH-Werten von 4 - 7,5 eines typischen feuchten oder feuchtnassen Wundmilieus kationisch geladen vorliegt und daher von negativen Gruppen des anionischen superabsorbierenden Materials attraktiv angezogen wird und so innerhalb des Saug-/Spülkörpers antimikrobiell wirkt. Das Flächengewicht des superabsorbierenden Materials innerhalb des Saug-/Spülkörpers beträgt vorzugsweise 30 - 150 g/m², insbesondere 50 - 100 g/m², insbesondere 60 - 80 g/m².

Nach einem weiteren Erfindungsgedanken von besonderer Bedeutung wird vorgeschlagen, dass die Dicke der Wundauflage im mit salzhaltiger wässrigen Lösung beaufschlagten Zustand 3 - 7 mm, insbesondere 4 - 6 mm beträgt.

Es wird hierbei davon ausgegangen, dass die Dicke der wundabgewandten Faservliesschicht und der darunter befindlichen Kunststofffilmschicht sowie die Dicke der wundzugewandten Schicht aus textilem Flächenmaterial mit der partiell und strukturiert aufgebrachten atraumatischen Beschichtung höchstens 1 mm der Gesamtdicke der herstellerseitig befeuchteten Wundauflage ausmachen, so dass der Rest von dem flüssigkeitsspeichernden Saug-/Spülkörper herrührt. Gegenüber vorbekannten Wundauflagen für die feuchtnasse Wundtherapie ist die wie vorstehend ausgebildete Wundauflage mit deutlich geringerer Dicke ausgebildet. Es wurde überraschenderweise festgestellt, dass das Absorptionsverhalten für Wundexsudate aber nicht herabgesetzt ist. Eine mögliche, aber noch nicht belegte Erklärung hierfür wäre, dass für die Absorption von Wundexsudat in dem mit salzhaltiger wässriger Lösung aktivierten Saug-/Spülkörper im Wesentlichen eine Oberflächenschicht des Saug-/Spülkörpers verantwortlich ist. Diese an sich überraschende Eigenschaft eröffnet die Möglichkeit, mit geringeren Flächengewichten der Komponenten des Saug-/Spülkörpers zu arbeiten, was wiederum die Dicke der Wundauflage insgesamt reduziert und ihre Biegsamkeit und Drapierfähigkeit verbessert.

Wie eingangs bereits erwähnt, kann das Verbinden der Schichten in vorteilhafter Weise durch Ultraschall- oder Laserschweißen durchgeführt werden.

Das flächige Trägermaterial überragt den Saug-/Spülkörper an allen Seiten, so dass ein allseitig den Saug-/Spülkörper umlaufender Rand gebildet wird, der auf der wundzugewandten Seite mit einer Schicht aus einem hautverträglichen Silikonklebstoff versehen ist. Eine derartig ausgestaltete Wundauflage wird auch als Inselverband oder "island dressing" bezeichnet.

Als hautverträgliche Silikonklebstoffe können insbesondere solche Silikonklebstoffe verwendet werden, die in K.L. Ulman, X. Thomas, "Silicone pressure sensitive adhesives for health-care applications", Advances in pressure sensitive technology-2 (1995), S. 133-157 beschrieben sind.

Durch die Verwendung von Silikonklebstoffen anstelle der sonst üblichen im Verbandmaterialbereich eingesetzten Klebstoffen, wie Naturkautschuk, Acrylatklebern, sowie sonstigen Hotmeltklebern besteht der Vorteil, dass sie zum einen ein Fixieren der Wundauflage erlauben, die Silikonklebstoffe neben ihrer hautfreundlichen Charakteristik darüber hinaus eine Hitzesterilisation erlauben, ohne dass der Kleber kollabiert und somit seine Klebekräfte einbüßt.

Dabei kann vorgesehen sein, dass als Trägermaterial ein solches Material eingesetzt wird, das im Bereich von 60 bis 150 °C hitzebeständig bzw. hitzestabil ist. Dabei kann es sich insbesondere um ein Folienmaterial oder ein textiles Material handeln, wobei Polyester, Polyurethan, Polypropylen oder Polyamid als geeignete Grundmaterialien eingesetzt werden können. Wird als Grundmaterial Polyurethan eingesetzt, können Polyether-basierte oder Polyester-basierte Polyurethane verwendet werden, wobei Polyether-basierte Polyurethane eine größere Thermostabilität und eine geringere Neigung zum Verzug unter thermischem Einfluss aufweisen und daher bevorzugt sind.

In einer bevorzugten Ausführungsform umfasst das Trägermaterial ein Folienmaterial aus Polester, Polyurethan, Polypropylen oder Polyamid, welches mit Öffnungen versehen ist. Die Öffnungen können jede geometrische Form aufweisen, beispielsweise Kreisform, Oval, Rechteck, Quadrat, Polygon. Die Öffnungen können einen effektiven Durchmesser von 0,1 - 10 mm aufweisen, bevorzugt 1 - 5 mm, besonders bevorzugt 1,5 - 3 mm. Die Öffnungen können unregelmäßig über die Folienfläche verteilt sein oder in regelmäßigen Mustern angeordnet sein. Die Öffnungen können einen insgesamt offenen Flächenanteil von 1 - 90 %, bevorzugt von 2 -75 %, besonders bevorzugt von 5 - 35%, ganz besonders bevorzugt von 10 - 20 % aufweisen. Ein geeignetes Material ist unter der Bezeichnung Acrysil^{™} der Firma Zodiac (Zodiac Coating, Pusignan, Frankreich) erhältlich.

In einer alternativ bevorzugten Ausführungsform umfasst das Trägermaterial ein textiles Material, welches Öffnungen aufweist. Geeignete Materialien sind in EP2561844 oder EP2561896 beschrieben. Bevorzugt handelt es sich um ein Gestrick oder Gewirk aus Polyesterfasern, welches so gefertigt ist, dass von den Maschen sehr kleine Öffnungen und dazwischen in einem regelmäßigen Muster angeordnete größere Öffnungen gebildet werden. Die sehr kleinen Öffnungen haben dabei einen mittleren Durchmesser von weniger als 1,0 mm, bevorzugt von weniger als 0,5 mm, besonders bevorzugt von weniger als 0,3 mm. Die größeren Öffnungen haben annähernd Kreis- oder Ovalform und einen mittleren Durchmesser von 1,5 - 5 mm, bevorzugt von 1,8 - 3,0 mm. Die Größe der Maschen ist dabei so gewählt, dass die sehr kleinen Öffnungen beim Beschichtungsvorgang mit Silikonklebstoff verschlossen werden, während die größeren Öffnungen unverschlossen bleiben. Ein geeignetes Material ist von der Firma Zodiac unter der Bezeichnung Novespire HB^{™} erhältlich (Zodiac Coating, Pusignan, Frankreich).

Die Verwendung hautfreundlicher Silikonklebstoffe auf einem Trägermaterial zur Fixierung einer Wundauflage auf der Haut eines Patienten geht mit einer deutlichen Verringerung der Wasserdampfdurchlässigkeit (MVTR) und Atmungsaktivität des beschichteten Materials einher. Eine ausreichende Wasserdampfdurchlässigkeit ist jedoch notwendig, um das Risiko einer Okklusion der mit der Silikonklebstoffschicht in Kontakt stehenden Haut zu verringern. Dazu ist es vorteilhaft, Öffnungen in der Trägerschicht vorzusehen, die durch den Silikonklebstoff nicht verschlossen werden.

Das Trägermaterial kann bevorzugt Öffnungen aufweisen, die von der aufgebrachten Schicht eines hautfreundlichen Silikonklebers nicht verschlossen werden.

In einer bevorzugten Ausführungsform weist das Trägermaterial ein mehrschichtiges Material auf mit wenigstens einer ersten und einer zweiten Schicht, wobei die erste Schicht Öffnungen aufweist, die nicht von Silikonklebstoff verschlossen sind, und die zweite Schicht vollflächig ausgestaltet ist.

Das Trägermaterial kann einen mehrschichtigen Verbund aus gleichartigen oder verschiedenartigen Materialien umfassen. Dabei besteht das Trägermaterial bevorzugt aus einer auf der wundabgewandten Seite angeordneten wasserdampfdurchlässigen und flüssigkeitsundurchlässigen Schicht, bevorzugt einer Polyurethanschicht mit einer Dicke von 10 - 100pm, bevorzugt 15 - 50µm, besonders bevorzugt 20 - 30 µm und einer mit Öffnungen versehenen Schicht aus einem Folienmaterial oder textilen Material auf der wundzugewandten Seite, welche wiederum auf der wundzugewandten Seite mit einem hautfreundlichen Silikonklebstoff beschichtet ist. Bevorzugt weist ein derartiges mit Silikonklebstoff beschichtetes Trägermaterial eine Wasserdampfdurchlässigkeit von mindestens 1000 g/m²/24h, insbesondere mindestens 2500 g/m²/24h, bevorzugt mindestens 5000 g/m²/24h auf, gemessen nach NF EN 13726-2 (inverted cup method).

Dabei können die einzelnen Lagen des mehrschichtigen Verbunds durch jedes geeignete Verfahren miteinander verbunden werden, beispielsweise durch Hitze, Druck, Klebstoff oder Kombinationen davon.

Dabei kann vorgesehen sein, dass die Trägerschicht in der mit Öffnungen versehenen Schicht in einem zentralen Bereich eine Aussparung in der Größe des Saug-/Spülkörpers aufweist.

Die auf der wundabgewandten Seite angeordnete wasserdampfdurchlässige und flüssigkeitsundurchlässige Schicht bedeckt dabei den Saug-/Spülkörper auf der wundabgewandten Seite. Bevorzugt wird in diesem Fall die Verbindung zwischen der wasserdampfdurchlässigen und flüssigkeitsundurchlässigen Schicht und dem Saug-/Spülkörper durch einen Klebstoff oder ein doppelseitiges Klebeband vermittelt.

Dabei kann generell vorgesehen sein, dass die Schicht aus hautfreundlichem Silikonklebstoff ("Klebeschicht") auf der wundzugewandten Seite vollflächig oder auch lediglich abschnittsweise, insbesondere in Mustern, aufgebracht ist. Durch einen Auftrag in Mustern kann die Wasserdampfdurchlässigkeit des Trägermaterials bei einem applizierten erfindungsgemäßen Wundverband verbessert werden. Damit kann dem Problem, dass die Wasserdampfdurchlässigkeit eines Wundverbandes im Bereich eines Kleberauftrags generell herabgesetzt ist und insbesondere die Silikonklebstoffe, die zwar hautverträglich sind, jedoch nur eine begrenzte Wasserdampfdurchlässigkeit aufweisen, entgegengewirkt werden.

Insbesondere kann vorteilhaft vorgesehen sein, dass die Klebeschicht eine Dicke von 40 bis 500 µm, vorzugsweise 100 bis 450 µm, und besonders bevorzugt 250 bis 450 µm besitzt. Besonders bevorzugt kann eine Dicke der Klebeschicht von 310 bis 440µm µm vorgesehen sein. Das Flächengewicht, also die Auftragsmenge, ist dabei vorzugsweise < 375 g/m², besonders bevorzugt 100 - 300 g/m², ganz besonders bevorzugt 150 - 250 g/m².

Des Weiteren kann vorgesehen sein, dass die Klebkraft eines entsprechenden Silikonklebstoffs auf einer als Bristol paper bekannten Unterlage mindestens 0,5 N/25mm, insbesondere mindestens1,0 N/25 mm und besonders bevorzugt mindestens1,5 N/25 mm entspricht, wobei hier als Messverfahren für die Klebkraft FINAT No.1 mit einem Abzugswinkel von 180° gegenüber Bristol-Papier bei einer Abzugsgeschwindigkeit von 300mm pro Minute eingesetzt wird.

Eine Vielzahl von Formen sind denkbar, sofern das Wundkissen am Wundverband festgelegt ist und über das Trägermaterial eine Fixierung an einem Träger erfolgen kann. Dabei ist es möglich, das Trägermaterial als beliebig gestaltete Netz- oder Lochstruktur bereitzustellen, wobei wichtig ist, dass das Trägermaterial das Wundkissen weit genug überspannt, so dass ein sicheres Fixieren des Wundkissens auf einer Wunde sichergestellt werden kann.

Insbesondere kann vorgesehen sein, dass das Wundkissen über einen Bereich der Klebeschicht an dem Trägermaterial festgelegt ist, wobei der Kleber der Klebeschicht auch in diesem Bereich vorzugsweise ein oder mehrere Silikonklebstoffe umfasst. Es ist dabei vorteilhaft, im Bereich des Kontaktes zwischen Wundkissen und Silikonklebstoff die Oberfläche des Silikonklebstoffs vor Zusammenfügen der einzelnen Teile einer Oberflächenaktivierung chemischer Gruppen zu unterziehen. Dabei können chemische und/oder physikalische Verfahren eingesetzt werden. Geeignete Verfahren umfassen Behandlungen einer oder beider Oberflächen mit Plasma, Hitze, Gas, Flüssigkeit oder Coronabehandlung. Besonders geeignete Verfahren sind in der WO2015/044532 offenbart. So wird die Haftkraft zwischen Wundkissen und Trägermaterial erhöht und das Risiko der Delamination vermindert.

Darüber hinaus kann vorgesehen sein, dass die wundzugewandte Seite des Klebers mit einer im Bereich von 60 bis 150 °C hitzestabilen Schutzschicht versehen ist.

Eine erfindungsgemäße Wundauflage weist eine erste und eine zweite Schutzschicht auf. Die erste Schutzschicht bedeckt die wundzugewandte Seite der auf einer Trägerschicht vorhandenen Lage eines Silikonklebstoffs. Sie schützt die Silikonklebstoffschicht vor ungewünschtem Kontakt mit Flüssigkeit, die aus dem mit salzhaltiger wässriger Lösung beaufschlagten Saug-/Spülkörper stammen kann. Sie schützt die Silikonklebstoffschicht außerdem vor Kontakt mit Wasserdampf, der beispielsweise während des Sterilisationsvorgangs auftreten kann. Die zweite Schutzschicht überdeckt die wundabgewandte Seite des Trägermaterials und schützt so die Silikonkleberschicht ebenfalls vor ungewünschtem Kontakt mit Flüssigkeit, die aus dem mit salzhaltiger wässriger Lösung beaufschlagten Saug-/Spülkörper stammen kann, und mit Wasserdampf. Bei der ersten und zweiten Schutzschicht kann es sich insbesondere um eine Folie, insbesondere eine nicht quellbare Polymerfolie, handeln. Die Abdeckung mit einer solchen Schutzschicht, die auch als Liner bezeichnet werden kann, besitzt den Vorteil, dass kein Anhaften des Wundverbandes, beispielsweise an einer Umverpackung, erfolgt und so ein leichteres Entnehmen des Wundverbandes aus der Umverpackung gewährleistet ist. Darüber hinaus gewährleisten beide Schichten gemeinsam, dass die Silikonklebstoffschicht des Trägermaterials nicht in Kontakt mit Flüssigkeit aus dem Saug-/Spülkörper und nicht in Kontakt mit Wasserdampf gerät.

Sowohl die erste als auch die zweite Schutzschicht überragen dabei das Trägermaterial ganzseitig.

Produktionstechnisch ist es vorteilhaft, in einem ersten Schritt zunächst eine vollständige trockene Wundauflage herzustellen, die anschließend in einem zweiten Schritt vollständig mit salzhaltiger wässriger Lösung beaufschlagt wird.

Sowohl die erste als auch die zweite Schutzschicht bestehen aus einem flüssigkeits- und wasserdampfundurchlässigen Material.

Dadurch wird sichergestellt, dass die Silikonklebstoffschicht des Trägermaterials nicht in Kontakt mit Flüssigkeit aus dem Saug-/Spülkörper und ebenso wenig in Kontakt mit Wasserdampf gerät, der insbesondere beim Sterilisationsvorgang in großer Menge entsteht. Ein Kontakt ist weder möglich von der wundzugewandten Seite aus noch über die Öffnungen oder die wasserdampfdurchlässige Lage der Trägerschicht. Dadurch wird gewährleistet, dass die Silikonklebstoffschicht zum Beginn der Anwendung ihre unverminderte Klebkraft entfalten kann.

Sowohl die erste als auch die zweite Schicht besteht aus einem Material, welches bei Temperaturen von 100 - 150°C hitzestabil ist. Geeignete Materialien umfassen Polyester, Polyamid, Poylpropylen. Besonders bevorzugt handelt es sich bei dem Material der ersten und der zweiten Schutzschicht um jeweils eine Folie aus Polyethylenterephthalat mit einer Dicke von 40-100µm und einem Flächengewicht von 80-100g/m². Dadurch wird sichergestellt, dass die Schutzschicht bei den beim Sterilisationsvorgang auftretenden Temperaturen ihre Funktion beibehält.

Die erste Schutzschicht enthält eine Öffnung, die den Saug-/Spülkörper unbedeckt lässt. Dadurch wird gewährleistet, dass der trockene Saug-/Spülkörper in prozesstechnisch vorteilhafter Weise mit salzhaltiger wässriger Lösung beaufschlagt werden kann.

Es wurde unerwartet festgestellt, dass es vorteilhaft ist, die erste Schutzschicht, zweite Schutzschicht und Trägerschicht einheitlich aus den gleichen Grundmaterialien herzustellen. Sofern eine nachfolgende thermische Behandlung, z.B. Hitze- oder Dampfsterilisation, durchgeführt wird, weisen die aus gleichen Grundmaterialien hergestellten Lagen gleiche oder ähnliche Hitzestabilitäten und Hitzeausdehnungskoeffizienten auf. Bei der Verwendung unterschiedlicher Grundmaterialien können unterschiedliche Hitzeausdehnungskoeffizienten zur Faltenbildung in den einzelnen Lagen führen. Das kann negative Auswirkungen auf die Applikationsfähigkeit, den Tragekomfort und/oder die Sicherheit vor dem Eindringen von Schmutz, Flüssigkeit, Gas oder Mikroorganismen haben.

Zur Applikation der erfindungsgemäßen Wundauflage wird zunächst die erste Schutzschicht entfernt. Der verbleibende Teil der Wundauflage wird auf die zu behandelnde Wunde aufgebracht. Anschließend wird die zweite Schutzschicht entfernt.

Die zweite Schutzschicht kann auf ihrer wundzugewandten Seite mit einem Klebstoff beschichtet sein. Das führt zu einer verstärkten Adhäsionskraft zwischen der Trägerschicht und der zweiten Schutzschicht, die die Adhäsionskraft zwischen der ersten Schutzschicht und der Silikonklebstoffschicht übertrifft, jedoch geringer ist als die Adhäsionskraft zwischen Haut des Patienten und Silikonklebstoffschicht. Auf diese Weise wird sichergestellt, dass bei Applikation der Wundauflage zunächst die erste Schutzschicht entfernt wird und erst anschließend die zweite Schutzschicht, während die Wundauflage am Patienten in der gewünschten Position fixiert bleibt. So kann eine hygienische Applikation an der gewünschten Stelle erzielt werden.

Der Klebstoffauftrag auf der wundzugewandten Seite der zweiten Schutzschicht kann vollflächig oder partiell, in Streifen, Mustern oder Punkten gestaltet sein.

In einer weiteren bevorzugten Ausführungsform ist die erste Schutzschicht einteilig ausgestaltet.

In einer bevorzugten Ausführungsform ist die zweite Schutzschicht zwei- oder mehrteilig ausgestaltet.

In einer besonders bevorzugten Ausführungsform weist die zweite Schutzschicht einen mittleren Bereich und wenigstens einen Randbereich auf, wobei der mittlere Bereich auf der wundzugewandten Seite wenigstens partiell mit einem Klebstoff versehen ist und der wenigstens eine Randbereich klebstofffrei ist. Auf diese Weise wird im Randbereich ein Griffelement geschaffen, welches das Ablösen der zweiten Schutzschicht vom Trägermaterial erleichtert. Dies kann in einfacher Weise dadurch realisiert werden, dass ein zusätzlicher Materialabschnitt im Randbereich auf die mit Klebstoff beschichtete wundzugewandte Seite der zweiten Schutzschicht aufgebracht wird. Dieser zusätzliche Materialabschnitt kann zusammen mit der Außenkante der zweiten Schutzschicht abschließen oder darüber hinausragen. Bevorzugt handelt es sich bei dem zusätzlichen Materialabschnitt um einen Folienabschnitt aus demselben Material wie die erste und zweite Schutzschicht. Zur besseren Erkennbarkeit kann der zusätzliche Materialabschnitt farblich abgegrenzt sein. Bevorzugt weist er eine blaue Farbe auf.

In einer bevorzugten Ausführungsform weist die zweite Schutzschicht wenigstens eine Trennungslinie auf. Die wenigstens eine Trennungslinie teilt die zweite Schutzschicht in wenigstens zwei Teile. Auf diese Weise kann erreicht werden, dass die zweite Schutzschicht nicht nur vom Rand aus von der Trägerschicht entfernt werden kann, sondern auch von der Trennungslinie aus. In einer besonders bevorzugten Ausführungsform handelt es sich bei der Trennungslinie um eine Trennungslinie mit Wellenform. Dies ermöglicht eine besonders gute Möglichkeit für den Anwender, die zweite Schutzschicht zu ergreifen und von der Trägerschicht abzulösen.

In einer bevorzugten Ausführungsform weist der mit einer Trennungslinie versehene Bereich der zweiten Schutzschicht ein- oder beiderseitig der Trennungslinie eine zusätzliche Schicht aus einem Folienmaterial auf, so dass die ein- oder beiderseitig der Trennungslinie befindlichen Abschnitte verstärkt werden, so dass diese Bereiche einfacher zu ergreifen sind. Dies resultiert für den Anwender in einer besseren Handhabbarkeit der gesamten Wundauflage. In einer bevorzugten Ausführungsform weist dieser durch ein zusätzliches Folienmaterial verstärkte Bereich eine von der übrigen Schutzschicht verschiedene Farbe, bevorzugt blau auf, so dass dieser Bereich sehr einfach zu erkennen ist. Als Materialien bieten sich Kunststofffolien an, bevorzugt ein mit der ersten und zweiten Schutzschicht identisches Material. Dabei kann die zusätzliche Schicht aus Folienmaterial über einen Klebstoff mit der wundabgewandten Seite der zweiten Schutzschicht verbunden sein, so dass eine Trennung beider Materialien mit in der normalen Anwendung auftretenden Kräften nicht erfolgt.

Es kann vorgesehen sein, dass die erste Schutzschicht auf der wundzugewandten Seite und die zweite Schutzschicht auf der wundabgewandten Seit über ein Verbindungselement miteinander verbunden werden. Die erste Schutzschicht ist hierbei an einer Randseite durch das Verbindungselement scharnierartig fest mit der zweiten Schutzschicht verbunden. Das Verbindungselement weist wenigstens zwei gegeneinander bewegliche Schenkel auf. Die Schenkel des scharnierartigen Verbindungselements sind jeweils auf den Innen- oder Außenseiten der beiden miteinander verbundenen ersten und zweiten Schutzschichten befestigt. Sowohl die erste als auch die zweite Schutzschicht haben eine größere Flächenausdehnung als die Trägerschicht und überragen sie ganzseitig. Sowohl die erste als auch die zweite Schutzschicht können an der dem Verbindungselement gegenüberliegenden Randseite jeweils eine zusätzliche Ausformung aufweisen, die als Griffelement dient.

Zur Applikation wird zunächst das Wundversorgungsprodukt gemäß dieser Ausführungsform am Griffelement gefasst, wobei eine Hand das Griffelement fasst, die andere Hand den die Trägerschicht überragenden Teil der zweiten Schutzschicht. Dann wird die erste Schutzschicht von der Trägerschicht abgelöst. Die erste Schutzschicht und zweite Schutzschicht bleiben mittels des scharnierartigen Elementes miteinander verbunden. Das zentral angeordnete feuchte Wundkissen kann nun auf einer zu versorgenden Wunde appliziert und fixiert werden, ohne dass das Wundkissen oder die Trägerschicht mit den Händen berührt werden. Nach erfolgter Applikation wird die zweite Schutzschicht von der Rückseite der Trägerschicht abgelöst.

Erfindungsgemäße Wundauflagen können in beliebiger Form hergestellt werden. Dabei weisen sowohl die geeigneten Saug-/Spülkörper, als auch die geeigneten Trägermaterialien, also auch die geeigneten ersten und zweiten Schutzschichten beliebige geometrische Formen auf, wie beispielsweise Quadrate, Rechtecke, Trapeze, Dreiecke, Fünfecke, Sechsecke, andere Vielecke, Kreise, Ovale, Ellipsen. Bevorzugt werden Wundauflagen eingesetzt, bei denen sowohl der Saug-/Spülkörper, als auch das Trägermaterial, als auch die erste und zweite Schutzschicht äquivalente geometrische Formen aufweisen, die sich lediglich in den Ausdehnungsmaßen unterscheiden. Bevorzugt werden derartige quadratische, rechteckige, kreisförmige und ovale Wundauflagen.

Überraschender Weise wurde festgestellt, dass das vollständige Beaufschlagen des trockenen Saug-/Spülkörpers in prozesstechnisch vorteilhafter Weise auch durch eine besondere Ausgestaltung der Verpackung vorteilhaft unterstützt werden kann. Deshalb ist auch eine Verpackung für eine Wundauflage Gegenstand der vorliegenden Erfindung, wobei es sich bei der Wundauflage um eine Wundauflage zur Wundbehandlung im feuchten oder feuchtnassen Milieu handelt, die als sogenannter Inselverband ausgestaltet ist. Die Wundauflage umfasst also einen Saug-/Spülkörper, der herstellerseitig mit einer salzhaltigen wässrigen Lösung beaufschlagt ist und ein flächiges Trägermaterial, welches auf der wundabgewandten Seite des Saug-/Spülkörpers angeordnet ist und den Saug-/Spülkörper so überragt, dass ein allseitig den Saug-/Spülkörper umlaufender Rand gebildet wird, der auf der wundzugewandten Seite mit einer Schicht aus einem hautverträglichen Klebstoff versehen ist. Ferner umfasst die Wundauflage eine Schutzschicht, die die Schicht aus einem hautverträglichen Klebstoff auf der wundzugewandten Seite abdeckt. Bevorzugt handelt es sich um eine erfindungsgemäße Wundauflage.

Eine vorteilhafte Verpackung für eine derartige Wundauflage zur Wundbehandlung im feuchten oder feuchtnassen Milieu umfasst einen Beutel mit einer ersten Beutelseite aus einem ersten flüssigkeitsundurchlässigen Material und einer zweiten Beutelseite aus einem zweiten flüssigkeitsundurchlässigen Material, welche umlaufend randseitig miteinander flüssigkeitsdicht verbunden sind. Die flüssigkeitsundurchlässigen Materialien der ersten und zweiten Beutelseite können identisch oder verschieden sein. Bevorzugt handelt es sich jeweils um Folien aus Aluminium-Kunststoff-Verbundmaterial, besonders um Aluminium, welches auf der Außenseite mit einer Schicht aus Polyester und auf der Innenseite mit einer Schicht aus Polypropylen versehen ist. Die erste Beutelseite weist einen zentralen Bereich und einen Randbereich auf, welcher den zentralen Bereich allseitig umgibt. Der zentrale Bereich weist eine Vertiefung auf, die zur Positionierung der Wundauflage in der Verpackung mittels ihres Saug-/Spülkörpers geformt ist. Die Vertiefung hat eine Form und Größe, die ausreichend ist, um den von der Wundauflage abstehenden Saug-/Spülkörper aufzunehmen, bevorzugt auch im vollständig mit salzhaltiger wässriger Lösung beaufschlagten Zustand vollständig aufzunehmen. Vorzugsweise hat die Vertiefung eine Form, die zu der Form des Saug-/Spülkörpers in trockenem Zustand komplementär ist. Vorzugsweise weist die Vertiefung eine Tiefe auf, die 50-150%, bevorzugt 60-140%, besonders bevorzugt 80-120% und ganz besonders bevorzugt 90-110% der Dicke des Saug-Spülkörpers in trockenem Zustand entspricht. Vorzugsweise weist die Vertiefung eine Längenausdehnung auf, die 100-300%, bevorzugt 100-200%, besonders bevorzugt 100-150%, ganz besonders bevorzugt 100-120% der Längenausdehnung des Saug-/Spülkörpers in trockenem Zustand entspricht. Vorzugsweise weist die Vertiefung eine Breitenausdehnung auf, die 100-300%, bevorzugt 100-200%, besonders bevorzugt 100-150%, ganz besonders bevorzugt 100-120% der Breitenausdehnung des Saug-/Spülkörpers in trockenem Zustand entspricht. Vorzugsweise weist die Vertiefung eine Form auf, die zu der Form des Saug-/Spülkörpers in trockenem Zustand komplementär ist, deren Volumen jedoch um 1-200%, bevorzugt 2-100%, besonders bevorzugt 3-75% größer ist als der vollständig mit salzhaltiger wässriger Lösung beaufschlagte Saug-/Spülkörper. In bevorzugten Ausführungsformen können die hier offenbarten Bereiche für Tiefe, Längenausdehnung, Breitenausdehnung und Volumen der Vertiefung beliebig miteinander kombiniert werden. In einer besonders bevorzugten Ausführungsform weist die Vertiefung eine Tiefe auf, die 90-110% der Dicke des Saug-/Spülkörpers in trockenem Zustand entspricht, sowie eine Längenausdehnung und Breitenausdehnung, die jeweils 100-120% der Längenausdehnung und Breitenausdehnung des Saug-/Spülkörpers in trockenem Zustand entspricht. Eine derartige Vertiefung kann für beliebige Beutelmaterialien in einem als Tiefziehverfahren bekannten Prozess hergestellt werden.

Weiterhin ist ein Gegenstand der vorliegenden Erfindung ein Verfahren zur Herstellung eines Wundversorgungsprodukts umfassend eine erfindungsgemäße Wundauflage und eine erfindungsgemäße Verpackung. Das Verfahren umfasst die folgenden Schritte a) Bereitstellen einer ersten Beutelseite aus einem ersten flüssigkeitsundurchlässigen Material, b) Bereitstellen einer Vertiefung in einem zentralen Bereich der ersten Beutelseite in einem Tiefziehverfahren, c) Befüllen der Vertiefung mit einer bestimmten Menge salzhaltiger wässriger Lösung, die zur vollständigen Beaufschlagung eines Saug-/Spülkörpers der Wundauflage geeignet ist, d) Einlegen einer Wundauflage gemäß einem der Ansprüche 1-11 auf die erste Beutelseite, wobei eine Positionierung der Wundauflage mittels ihres Saug-/Spülkörpers in der Vertiefung erfolgt, e) Auflegen einer zweiten Beutelseite aus einem zweiten flüssigkeitsundurchlässigen Material, f) flüssigkeitsdichtes Versiegeln der ersten und zweiten Beutelseite entlang einer die gesamte Wundauflage allseitig umlaufenden Verbindungslinie, und g) Sterilisation.

In einem Produktionsprozess wird zunächst die erste Beutelseite mit einer im Tiefziehverfahren hergestellten Vertiefung bereitgestellt. In die Vertiefung wird eine erforderliche Menge salzhaltiger wässriger Lösung eingefüllt. Bevorzugt ist diese Menge ausreichend, um den Saug-/Spülkörper vollständig, also bis zur Sättigung, mit salzhaltiger wässriger Lösung zu beaufschlagen. Anschließend wird eine erfindungsgemäße Wundauflage auf die erste Beutelseite aufgelegt, wobei die wundzugewandte Seite der Wundauflage in Richtung der ersten Beutelseite weist. Dabei wird der Saug-/Spülkörper der Wundauflage in die mit salzhaltiger wässriger Lösung befüllte Vertiefung der ersten Beutelseite eingeführt. Die enthaltene salzhaltige wässrige Lösung wird dabei vom Saug-/Spülkörper vollständig absorbiert. Anschließend wird eine zweite Beutelseite so auf der ersten Beutelseite platziert, dass die auf der ersten Beutelseite aufliegende Wundauflage vollständig von der zweiten Beutelseite bedeckt ist. Die beiden Beutelseiten werden miteinander so verbunden, dass eine Siegelnaht gebildet wird, welche die Wundauflage allseitig umgibt. An wenigstens einer Seite ragt jeweils wenigstens ein Teil der ersten und zweiten Beutelseite über die an dieser Seite vorhandene Siegelnaht hinaus. So können diese beiden die Siegelnaht überragenden Beutelseitenteile mit jeweils einer Hand gegriffen werden und auseinandergezogen werden, so dass die Siegelnähte geöffnet werden, damit die in der Verpackung platzierte Wundauflage entnommen und am Patienten appliziert werden kann.

Zur Sterilisation der erfindungsgemäßen Wundauflage kann eine Wasserdampfsterilisation mit Gegendruck verwendet werden. Hierbei können Temperaturen von 90 - 130 °C verwendet werden.

Eine Kollabierung des Klebstoffes fand nicht statt.

Weiterhin ist Gegenstand der vorliegenden Erfindung ein Sortiment zur Verwendung in der Wundversorgung umfassend verschiedene Wundauflagen. Ein derartiges Sortiment enthält mindestens eine erste erfindungsgemäße Wundauflage und wenigstens eine zweite erfindungsgemäße Wundauflage. Unter einem Sortiment wird ein Angebot mehrerer unterschiedlicher Wundauflagen verstanden, die nicht notwendiger Weise in gemeinsamen Verkaufseinheiten angeboten werden müssen, sondern beispielsweise in einem gemeinsamen Angebot in voneinander getrennten Verkaufseinheiten. Dabei weist die erste Wundauflage einen ersten Saug-/Spülkörper mit einer Dicke und einer ersten geometrischen Form auf und die zweite Wundauflage einen Saug-/Spülkörper mit einer Dicke und einer zweiten geometrischen Form. In einem derartigen Sortiment ist die Dicke des ersten Saug-/Spülkörpers mit der Dicke des zweiten Saug-/Spülkörpers identisch und die erste geometrische Form von der zweiten geometrischen Form verschieden. Die Bereitstellung eines Sortiments von Wundauflagen mit verschiedenen geometrischen Formen hat den Vorteil, dass für verschiedene Anwendungssituationen geeignete Wundauflagen zur Verfügung gestellt werden können. Dabei weisen Wundauflagen in unterschiedlicher geometrischer Form beispielsweise Vorteile bei der Applikation an verschiedenen Körperstellen auf. Die Bereitstellung eines Sortiments von Wundauflagen mit Saug-/Spülkörpern mit unterschiedlicher geometrischer Form aber identischer Dicke hat auf der einen Seite produktionstechnische Vorteile, weil dasselbe Ausgangs- oder Zwischenmaterial für den Saug-/Spülkörper für die Herstellung geometrisch verschieden gestalteter Wundauflagen verwendet werden kann. Zum anderen hat sich, wie eingangs erwähnt, die Dicke des Saug-/Spülkörpers als vorteilhaft für den Patienten hinsichtlich des Tragekomforts erwiesen.

Weiterhin soll die Erfindung im Folgenden anhand einer Zeichnung näher erläutert werden. Dabei zeigt
Figur 1 eine schematisch dargestellte erfindungsgemäße Wundauflage in Draufsicht,
Figur 2 eine schematisch dargestellte erfindungsgemäße Wundauflage im Querschnitt,
Figur 3 eine schematisch dargestellte erfindungsgemäße Wundauflage in einer alternativen Ausführungsform im Querschnitt.
Figur 4 zeigt eine schematisch dargestellte Verpackung für eine erfindungsgemäße Wundauflage im Querschnitt.

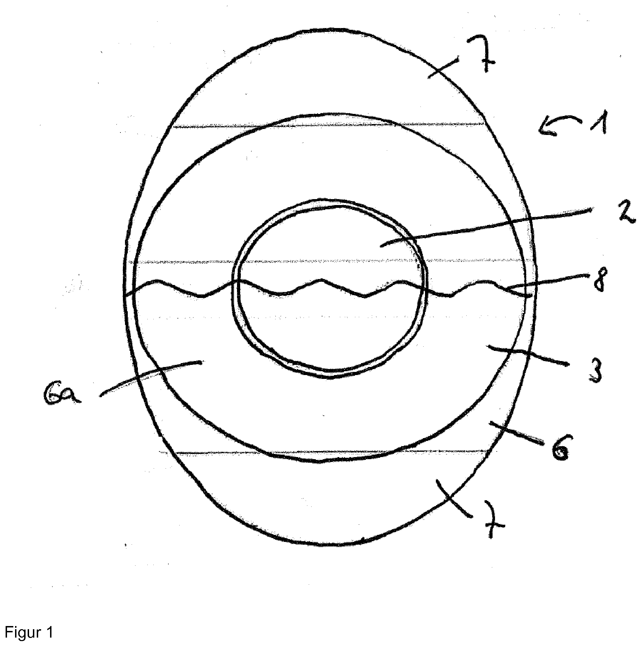

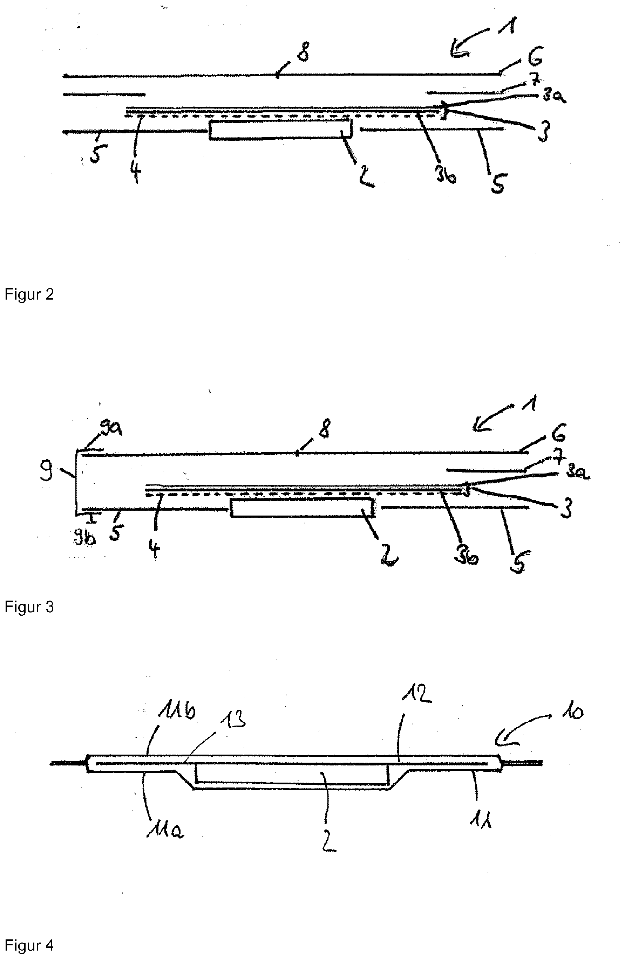

Die Figuren 1 und 2 zeigen eine erfindungsgemäße Wundauflage (1) umfassend einen Saug-/Spülkörper (2), der herstellerseitig mit Ringerlösung beaufschlagt ist und ein flächiges Trägermaterial (3) auf der wundabgewandten Seite des Saug-/Spülkörpers (2). Das flächige Trägermaterial (3) ist ein zweischichtiges Material umfassend eine flüssigkeitsundurchlässige und wasserdampfdurchlässige äußere Schicht (3a) aus einem Polyurethan und eine perforierte Polyurethanfolie (3b) auf der wundzugewandten Seite. Die Öffnungen der perforierten Polyurethanfolie (3b) haben eine annähernd kreisförmige Form. Auf der wundzugewandten Seite ist die Polyurethanfolie (3b) mit einem hautverträglichen Silikonklebstoff (4) versehen. Dabei werden die Öffnungen der Folienschicht durch den Silikonklebstoff nicht verschlossen.

Die Wundauflage (1) weist auf der wundzugewandten Seite eine erste, einteilige Schutzschicht (5) aus Polyesterfolie in einer Dicke von 50µm auf.

Die Wundauflage (1) weist auf der wundabgewandten Seite eine zweite Schutzschicht (6) auf, die vollflächig mit einem Acrylatkleber beschichtet ist. Die zweite Schutzschicht weist an zwei Rändern Griffelemente (7) auf. Diese Griffelemente (7) werden aus Materialabschnitten aus Polyethylenterephthalat gebildet, die auf der wundzugewandten Seite der zweiten Schutzschicht (6) aufgebracht und über den Acrylatkleber mit der zweiten Schutzschicht (6) verbunden sind. Die Griffelemente (7) sind farblich von der zweiten Schutzschicht (6) abgesetzt. Die zweite Schutzschicht (6) ist in der Mitte durch eine schlangenförmig ausgestaltete Trennlinie (8) in zwei Teile getrennt.

Figur 3 zeigt eine erfindungsgemäße Wundauflage als weitere Ausgestaltung der in Figur 2 beschriebenen Wundauflage. In dieser Ausführungsform sind die erste und zweite Schutzschicht (5, 6) an einer Randseite durch ein Verbindungselement (9) scharnierartig miteinander fest verbunden sind, wobei die Schenkel (9a, 9b) des scharnierartigen Verbindungselements (9) jeweils auf den Außenseiten der beiden miteinander verbundenen ersten und zweiten Schutzschicht (5,6) befestigt sind.

Figur 4 zeigt eine erfindungsgemäße Verpackung (10) mit einer Wundauflage (12). Bei der Wundauflage handelt es sich um eine Wundauflage (12) zur Wundbehandlung im feuchten oder feuchtnassen Milieu, die als sogenannter Inselverband ausgestaltet ist. Die Wundauflage (12) umfasst a) einen Saug-/Spülkörper (2), der herstellerseitig mit einer salzhaltigen wässrigen Lösung beaufschlagt ist, b) ein flächiges Trägermaterial (3), welches auf der wundabgewandten Seite des Saug-/Spülkörpers (2) angeordnet ist und den Saug-/Spülkörper (2) so überragt, dass ein allseitig den Saug-/Spülkörper (2) umlaufender Rand gebildet wird, der auf der wundzugewandten Seite mit einer Schicht aus einem hautverträglichen Klebstoff versehen ist, und c) eine Schutzschicht, die die Schicht aus einem hautverträglichen Klebstoff auf der wundzugewandten Seite abdeckt. Die Verpackung wird durch einen Beutel (11) aus einem ersten Materialabschnitt (11a) und einem zweiten Materialabschnitt (11b) gebildet. Bei dem ersten und dem zweiten Materialabschnitt handelt es sich jeweils um Folien aus mit auf der Außenseite mit Polyester und auf der Innenseite mit Polypropylen beschichtetem Aluminium. Der erste Materialabschnitt (11a) weist eine Vertiefung auf, deren Form zu der des Saug-/Spülkörpers (2) komplementär ist und eine Größe aufweist, die geeignet ist, einen Saug-/Spülkörper (2) einer enthaltenen Wundauflage (12) aufzunehmen.

## Patentansprüche

1. Wundauflage (1) zur Wundbehandlung im feuchten oder feuchtnassen Milieu umfassend a) einen Saug-/Spülkörper (2), der herstellerseitig mit einer salzhaltigen wässrigen Lösung, insbesondere Ringerlösung beaufschlagt ist, wobei die Lösung vorzugsweise eine Substanz mit antimikrobieller Wirkung enthält, b) ein flächiges Trägermaterial (3), welches auf der wundabgewandten Seite des Saug-/Spülkörpers (2) angeordnet ist und den Saug-/Spülkörper (2) so überragt, dass ein allseitig den Saug-/Spülkörper (2) umlaufender Rand gebildet wird, der auf der wundzugewandten Seite mit einer Schicht aus einem hautverträglichen Silikonklebstoff (4) versehen ist, und c) einer ersten Schutzschicht (5), die die Schicht aus einem hautverträglichen Silikonklebstoff (4) auf der wundzugewandten Seite abdeckt,
**dadurch gekennzeichnet, dass** die Wundauflage (1) eine zweite Schutzschicht (6) aufweist, die auf der wundabgewandten Seite des Trägermaterials (3) angeordnet ist, wobei sowohl die erste als auch die zweite Schutzschicht (5, 6) das Trägermaterial (3) ganzseitig überragen, wobei die erste Schutzschicht (5) eine Öffnung bildet, die den Saug-/Spülkörper (2) unbedeckt lässt und wobei die erste und zweite Schutzschicht (5, 6) aus einem flüssigkeits- und wasserdampfundurchlässigen Material besteht, welches Polyester, Polyamid oder Polypropylen umfasst, so dass die erste und zweite Schutzschicht (5, 6) die Schicht aus hautverträglichem Silikonklebstoff (4) vor Kontakt mit Flüssigkeit und Wasserdampf schützt.

2. Wundauflage (1) nach Anspruch 1 **dadurch gekennzeichnet, dass** das Trägermaterial (3) eine Lage (3b) aus einem Material aufweist, welches Öffnungen enthält, die nicht von Silikonklebstoff verschlossen sind.

3. Wundauflage (1) nach mindestens einem der vorgenannten Ansprüche, **dadurch gekennzeichnet, dass** das Trägermaterial (3) eine Kunststofffolie oder ein textiles Material umfasst.

4. Wundauflage (1) gemäß einem der vorangehenden Ansprüche, **dadurch gekennzeichnet, dass** das Trägermaterial (3) mehrere Schichten (3a, 3b) umfasst mit wenigstens einer ersten (3b) und einer zweiten (3a) Schicht, wobei die erste Schicht (3b) Öffnungen aufweist, die nicht von Silikonklebstoff verschlossen sind, und die zweite Schicht (3a) durchgehend ausgestaltet ist.

5. Wundauflage (1) gemäß einem der vorangehenden Ansprüche, **dadurch gekennzeichnet, dass** die erste und zweite Schutzschicht (5, 6) aus Polyester besteht.

6. Wundauflage (1) gemäß einem der vorangehenden Ansprüche, **dadurch gekennzeichnet, dass** die zweite Schutzschicht (6) zwei- oder mehrteilig ausgestaltet ist.

7. Wundauflage (1) gemäß einem der vorangehenden Ansprüche, **dadurch gekennzeichnet, dass** die zweite Schutzschicht (6) einen mittleren Bereich und wenigstens einen Randbereich aufweist, wobei der mittlere Bereich auf der wundzugewandten Seite wenigstens partiell mit einem Klebstoff versehen ist und der wenigstens eine Randbereich klebstofffrei ist.

8. Wundauflage (1) gemäß Anspruch 7, **dadurch gekennzeichnet, dass** der wenigstens eine Randbereich einen zusätzlichen Materialabschnitt (7) umfasst, der untrennbar mit dem mittleren Bereich verbunden ist.

9. Wundauflage (1) gemäß einem der vorangehenden Ansprüche, **dadurch gekennzeichnet, dass** die zweite Schutzschicht (6) eine Trennungslinie (8) aufweist.

10. Wundauflage (1) gemäß einem der vorangehenden Ansprüche, **dadurch gekennzeichnet, dass** die erste und die zweite Schutzschicht (5, 6) an einer Randseite durch ein Verbindungselement (9) scharnierartig miteinander fest verbunden sind, wobei das Verbindungselement (9) wenigstens zwei gegeneinander bewegliche Schenkel (9a, 9b) aufweist und die Schenkel (9a, 9b) des scharnierartigen Verbindungselements (9) jeweils auf den Innen- oder Außenseiten der beiden miteinander verbundenen ersten und zweiten Schutzschicht (5,6) befestigt sind.

11. Verfahren zur Herstellung eines Wundversorgungsprodukts umfassend eine Wundauflage (1) gemäß einem der Ansprüche 1-10, wobei das Verfahren die folgenden Schritte umfasst a) Bereitstellen einer ersten Beutelseite (11a) aus einem ersten flüssigkeitsundurchlässigen Material, b) Bereitstellen einer Vertiefung in einem zentralen Bereich der ersten Beutelseite (11a) in einem Tiefziehverfahren, c) Befüllen der Vertiefung mit einer bestimmten Menge salzhaltiger wässriger Lösung, die vorzugsweise zur vollständigen Beaufschlagung eines Saug-/Spülkörpers (2) der Wundauflage (1) geeignet ist, d) Einlegen einer Wundauflage (1) gemäß einem der Ansprüche 1-10 auf die erste Beutelseite (11a), wobei eine Positionierung der Wundauflage (1) mittels ihres Saug-/Spülkörpers (2) in der Vertiefung erfolgt, e) Auflegen einerzweiten Beutelseite (11b) aus einem zweiten flüssigkeitsundurchlässigen Material, f) flüssigkeitsdichtes Versiegeln der ersten und zweiten Beutelseite (11a, 11b) entlang einer die gesamte Wundauflage (1) allseitig umlaufenden Verbindungslinie, und g) Sterilisation.

12. Sortiment zur Verwendung in der Wundversorgung umfassend mindestens eine erste Wundauflage (1) gemäß einem der Ansprüche 1-10 und wenigstens eine zweite Wundauflage (1) gemäß einem der Ansprüche 1-10, wobei die erste Wundauflage (1) gemäß einem der Ansprüche 1-10 einen ersten Saug-/Spülkörper (2) mit einer Dicke und einer ersten geometrischen Form aufweist und die zweite Wundauflage (1) gemäß einem der Ansprüche 1-10 einen Saug-/Spülkörper (2) mit einer Dicke und einer zweiten geometrischen Form aufweist, **dadurch gekennzeichnet, dass** die Dicke des ersten Saug-/Spülkörpers (2) mit der Dicke des zweiten Saug-/Spülkörpers (2) identisch ist und dass die erste geometrische Form von der zweiten geometrischen Form verschieden ist.

## Claims

1. Wound dressing (1) for wound treatment in a damp or moist environment, comprising a) a suction/rinsing body (2) treated by the manufacturer with a saline aqueous solution, in particular Ringer's solution, wherein the solution preferably contains a substance with antimicrobial action; b) a sheetlike backing material (3), which is arranged on that side of the suction/rinsing body (2) facing away from the wound and which overhangs the suction/rinsing body (2) in such a way as to form an edge, which surrounds the suction/rinsing body (2) on all sides and which, on the wound-facing side, is provided with a layer of a skin-compatible silicone adhesive (4); and c) a first protective layer (5), which covers the layer of a skin-compatible silicone adhesive (4) on the wound-facing side,
**characterized in that** the wound dressing (1) has a second protective layer (6), which is arranged on that side of the backing material (3) facing away from the wound, wherein both the first and the second protective layer (5, 6) overhang the backing material (3) over the entire side, wherein the first protective layer (5) forms an opening which leaves the suction/rinsing body (2) uncovered, and wherein the first and the second protective layer (5, 6) consist of a material which is impermeable to liquid and water vapour and comprises polyester, polyamide or polypropylene, with the result that the first and the second protective layer (5, 6) protect the layer of skin-compatible silicone adhesive (4) from contact with liquid and water vapour.

2. Wound dressing (1) according to Claim 1, **characterized in that** the backing material (3) has a ply (3b) of a material containing openings which are not obturated by silicone adhesive.

3. Wound dressing (1) according to at least either of the preceding claims, **characterized in that** the backing material (3) comprises a plastic film or a textile material.

4. Wound dressing (1) according to one of the preceding claims, **characterized in that** the backing material (3) comprises multiple layers (3a, 3b), with at least one first layer (3b) and one second layer (3a), wherein the first layer (3b) has openings which are not obturated by silicone adhesive, and the second layer (3a) is continuous.

5. Wound dressing (1) according to one of the preceding claims, **characterized in that** the first and the second protective layer (5, 6) consist of polyester.

6. Wound dressing (1) according to one of the preceding claims, **characterized in that** the second protective layer (6) has a two-part or multi-part form.

7. Wound dressing (1) according to one of the preceding claims, **characterized in that** the second protective layer (6) has a middle region and at least one edge region, wherein the middle region is at least partially provided with an adhesive on the wound-facing side, and the at least one edge region is adhesive-free.

8. Wound dressing (1) according to Claim 7, **characterized in that** the at least one edge region comprises an additional material portion (7) which is connected inseparably to the middle region.

9. Wound dressing (1) according to one of the preceding claims, **characterized in that** the second protective layer (6) has a separating line (8).

10. Wound dressing (1) according to one of the preceding claims, **characterized in that** the first and the second protective layer (5, 6) are fixedly connected to one another at one edge side in a hinge-like manner by a connecting element (9), wherein the connecting element (9) has at least two legs (9a, 9b) which can move with respect to one another, and the legs (9a, 9b) of the hinge-like connecting element (9) are secured respectively on the inner or outer sides of the two, first and second, protective layers (5, 6) connected to one another.

11. Method for producing a wound care product comprising a wound dressing (1) according to one of Claims 1-10, wherein the method comprises the following steps: a) providing a first pouch side (11a) of a first material which is impermeable to liquid, b) providing a depression in a central region of the first pouch side (11a) in a thermoforming process, c) filling the depression with a defined amount of saline aqueous solution preferably suitable for fully treating a suction/rinsing body (2) of the wound dressing (1), d) placing a wound dressing (1) according to one of Claims 1-10 onto the first pouch side (11a), wherein the wound dressing (1) is positioned by means of its suction/rinsing body (2) in the depression, e) laying-on a second pouch side (11b) of a second material which is impermeable to liquid, f) fluid-tightly sealing the first and the second pouch side (11a, 11b) along a connecting line surrounding the entire wound dressing (1) on all sides, and g) sterilizing.

12. Kit for use in wound care, comprising at least one first wound dressing (1) according to one of Claims 1-10 and at least one second wound dressing (1) according to one of Claims 1-10, wherein the first wound dressing (1) according to one of Claims 1-10 has a first suction/rinsing body (2) with a thickness and a first geometric shape, and the second wound dressing (1) according to one of Claims 1-10 has a suction/rinsing body (2) with a thickness and a second geometric shape, **characterized in that** the thickness of the first suction/rinsing body (2) is identical to the thickness of the second suction/rinsing body (2) and **in that** the first geometric shape is different from the second geometric shape.

## Revendications

1. Pansement (1) pour le traitement de plaies en milieu humide ou mouillé, comprenant a) un corps d'aspiration/de rinçage (2), qui est chargé par le fabricant avec une solution aqueuse saline, notamment une solution de Ringer, la solution contenant de préférence une substance à effet antimicrobien, b) un matériau support plat (3), qui est agencé sur le côté détourné de la plaie du corps d'aspiration/de rinçage (2) et qui dépasse du corps d'aspiration/de rinçage (2) de manière à former un bord entourant de tous côtés le corps d'aspiration/de rinçage (2), qui est pourvu sur le côté tourné vers la plaie d'une couche en un adhésif silicone (4) compatible avec la peau, et c) une première couche de protection (5) qui recouvre la couche en un adhésif silicone (4) compatible avec la peau sur le côté tourné vers la plaie,
**caractérisé en ce que** le pansement (1) présente une deuxième couche de protection (6) qui est agencée sur le côté du matériau support (3) détourné de la plaie, la première et la deuxième couche de protection (5, 6) dépassant toutes les deux du matériau support (3) de tous côtés, la première couche de protection (5) formant une ouverture, qui laisse le corps d'absorption/de rinçage (2) non recouvert et la première et la deuxième couche de protection (5, 6) étant constituées d'un matériau imperméable aux liquides et à la vapeur d'eau, qui comprend du polyester, du polyamide ou du polypropylène, de telle sorte que la première et la deuxième couche de protection (5, 6) protègent la couche en adhésif silicone (4) compatible avec la peau d'un contact avec les liquides et la vapeur d'eau.

2. Pansement (1) selon la revendication 1, **caractérisé en ce que** le matériau support (3) présente une strate (3b) en un matériau qui contient des ouvertures qui ne sont pas fermées par de l'adhésif silicone.

3. Pansement (1) selon au moins l'une quelconque des revendications précédentes, **caractérisé en ce que** le matériau support (3) comprend un film plastique ou un matériau textile.

4. Pansement (1) selon l'une quelconque des revendications précédentes, **caractérisé en ce que** le matériau support (3) comprend plusieurs couches (3a, 3b) avec au moins une première (3b) et une deuxième (3a) couche, la première couche (3b) présentant des ouvertures qui ne sont pas fermées par de l'adhésif silicone et la deuxième couche (3a) étant réalisée sous forme continue.

5. Pansement (1) selon l'une quelconque des revendications précédentes, **caractérisé en ce que** la première et la deuxième couche de protection (5, 6) sont constituées de polyester.

6. Pansement (1) selon l'une quelconque des revendications précédentes, **caractérisé en ce que** la deuxième couche de protection (6) est conçue en deux parties ou plus.

7. Pansement (1) selon l'une quelconque des revendications précédentes, **caractérisé en ce que** la deuxième couche de protection (6) présente une zone centrale et au moins une zone de bord, la zone centrale étant pourvue au moins partiellement d'un adhésif sur le côté tourné vers la plaie et l'au moins une zone de bord étant exempte d'adhésif.

8. Pansement (1) selon la revendication 7, **caractérisé en ce que** l'au moins une zone de bord comprend une section de matériau supplémentaire (7) qui est reliée de manière inséparable à la zone centrale.

9. Pansement (1) selon l'une quelconque des revendications précédentes, **caractérisé en ce que** la deuxième couche de protection (6) présente une ligne de séparation (8).

10. Pansement (1) selon l'une quelconque des revendications précédentes, **caractérisé en ce que** la première et la deuxième couche de protection (5, 6) sont reliées solidement l'une à l'autre à la manière d'une charnière sur un côté de bord par un élément de liaison (9), l'élément de liaison (9) présentant au moins deux branches (9a, 9b) mobiles l'une par rapport à l'autre et les branches (9a, 9b) de l'élément de liaison (9) à la manière d'une charnière étant fixées respectivement sur les côtés intérieurs ou extérieurs des deux première et deuxième couches de protection (5, 6) reliées entre elles.

11. Procédé de fabrication d'un produit de traitement de plaies comprenant un pansement (1) selon l'une quelconque des revendications 1 à 10, le procédé comprenant les étapes suivantes : a) la fourniture d'un premier côté de poche (11a) en un premier matériau imperméable aux liquides, b) la fourniture d'une cavité dans une zone centrale du premier côté de poche (11a) dans un procédé d'emboutissage profond, c) le remplissage de la cavité avec une quantité déterminée de solution aqueuse saline, qui est de préférence appropriée pour le chargement complet d'un corps d'aspiration/de rinçage (2) du pansement (1), d) l'insertion d'un pansement (1) selon l'une quelconque des revendications 1 à 10 sur le premier côté de poche (11a), un positionnement du pansement (1) étant effectué au moyen de son corps d'absorption/de rinçage (2) dans la cavité, e) la mise en place d'un deuxième côté de poche (11b) en un deuxième matériau imperméable aux liquides, f) le scellement étanche aux liquides du premier et du deuxième côté de poche (11a, 11b) le long d'une ligne de liaison entourant de tous côtés l'ensemble du pansement (1), et g) la stérilisation.

12. Assortiment pour utilisation dans le traitement de plaies, comprenant au moins un premier pansement (1) selon l'une quelconque des revendications 1 à 10 et au moins un deuxième pansement (1) selon l'une quelconque des revendications 1 à 10, le premier pansement (1) selon l'une quelconque des revendications 1 à 10 présentant un premier corps d'absorption/de rinçage (2) ayant une épaisseur et une première forme géométrique, et le deuxième pansement (1) selon l'une quelconque des revendications 1 à 10 présentant un corps d'absorption/de rinçage (2) ayant une épaisseur et une deuxième forme géométrique, **caractérisé en ce que** l'épaisseur du premier corps d'aspiration/de rinçage (2) est identique à l'épaisseur du deuxième corps d'aspiration/de rinçage (2) et **en ce que** la première forme géométrique est différente de la deuxième forme géométrique.
